# EUROPÄISCHE PATENTSCHRIFT

(11) **EP 0 812 858 B1**
(45) Veröffentlichungstag und Bekanntmachung des Hinweises auf die Patenterteilung: **27.09.2006**
(21) Anmeldenummer: 97109314.1
(22) Anmeldetag: 09.06.1997
(51) Int. Cl.: C07K 14/755

(54) **Verfahren zur Herstellung einer Faktor VIII-enthaltenden Zusammensetzung**
Process for the preparation of a composition containing factor VIII
Procédé pour la préparation d'une composition contenant le facteur VIII

(30) Priorität: 11.06.1996 DE 19623293
(43) Veröffentlichungstag der Anmeldung: 17.12.1997
(73) Patentinhaber: BIOTEST PHARMA GMBH, D-63303 Dreieich (DE)
(72) Erfinder: Bucci, Enzo, Dr., 02010 S. Rufina Cittaducale, Rieti (IT); Kotitschke, Ronald, Dr., 63303 Dreieich (DE); Rudnick, Dieter, Dr., 63303 Dreieich (DE); Dichtelmüller, Herbert, Dr., 65843 Sulzbach/Ts. (DE); Kloft, Michael, Dr., 64291 Darmstadt (DE); Loebnau, Wolfgang, 63533 Mainhausen (DE); Ott, Günter, 63110 Rodgau (DE)
(74) Vertreter: Keller, Günter

(56) Entgegenhaltungen:
- EP-A- 0 094 611
- EP-A- 0 567 448
- WO-A-94/17834
- THROMBOSIS AND HAEMOSTASIS 74 (3). 1995. 868-873, XP002040293 ARRIGHI S ET AL: ""In vitro" and in animal model studies on a double virus -inactivated factor VII concentrate."
- 39TH ANNUAL MEETING OF THE SOCIETY FOR THROMBOSIS AND HEMOSTASIS RESEARCH, BERLIN, GERMANY, FEBRUARY 15-18, 1995. ANNALS OF HEMATOLOGY 70 (SUPPL. 1). 1995. A35, XP002040294 ELODI S ET AL: "Double virus inactivated F VIII concentrate: Virus validation and clinical efficacy of Haemoctin (Biotest F VIII SDH)."
- HAEMOSTASEOLOGIE (STUTTGART) (1994), 14(2), 100-3, 1994, XP002040295 KOTITSCHKE, R. ET AL: "Double virus -inactivated factor VIII concentrate. Virus inactivation and validation of FVIII SDH"
- BIOLOGICALS 24 (2). 1996. 125-130, XP002040296 DICHTELMUELLER H ET AL: "Improvement of virus safety of a S-D-treated factor VIII concentrate by additional dry heat treatment at 100 degrees C."

## Beschreibung

Die Erfindung betrifft ein Verfahren zur Herstellung einer insbesondere hochgereinigten, doppelt virusinaktivierten Faktor VIII (Blutgerinnungs-Faktor VIII; abgek. "FVIII")-enthaltenden Zusammensetzung aus dem Blutplasma vom Menschen.

Im Stand der Technik sind Verfahren zur Herstellung hochreiner, virusinaktivierter FVIII-Produkte, die aus dem Blutplasma vom Menschen isoliert werden, beschrieben. Beispielsweise beschreibt EP O 367 840 die Herstellung eines FVIIIProduktes, das lonenaustauscher zur FVIII Reinigung verwendet. Zur Virusin-aktivierung wird die FVIII-Lösung mit biokompatiblen organischen Lösungsmitteln/Detergenzien behandelt. DE 42 04 694 beschreibt Anionenaustauscher mit verschiedenen Merkmalen, die zur FVIII Reinigung geeignet sind.
EP 0 337 144 beschreibt Trennmaterialien zur Fraktionierung von Biopolymeren, die bei der Affinitäts- oder Ionenaustauscher-Chromatographie verwendet werden können. EP O 567 448 beschreibt die Kombination der Maßnahmen einer chromatographischen Reinigung, einer Tensidbehandlung des FVIII in wäßriger Lösung und einer Hitzebehandlung der FVIII-Präparation in festem Zustand. Die Hitzebehandlung erfolgt mit heißem Dampf in Gegenwart von Methanol oder Ethanol unter Angabe bestimmter Mengenverhältnisse. WO 93/15105 beschreibt die FVIII-Herstellung aus Kryopräzipitat unter Verwendung von Anionenaustauschern. EP O 399 321 beschreibt die Herstellung eines FVIII-Konzentrates durch Ausfällung des FVIII mit Polyethylenglykol (PEG) und Wärmebehandlung in Gegenwart von Saccharose (1,2 bis 0,6 g/m FVlll-Konzentrat). FBE 0 383 645 beschreibt die Herstellung eines FVlll-Konzentrates durch Trennung des FVIII vom von Willebrand Faktor. EP 0 412 466 beschreibt die Herstellung von FVIII mit hoher spezifischer Aktivität, der zusätzlich pasteurisiert wird. US-Patent 5,099,002 beschreibt das Erhitzen von gefriergetrockneten Gerinnungskonzentraten zur Reduzierung der Infektiosität von Viren, falls vorhanden. WO 94/17 834 beschreibt die Inaktivierung von Viren durch Behandlung der Quelle mit Alkylphosphaten, gieichzeitig oder aufeinanderfolgend, und bei einer Temperatur von 55 °C bis 67 °C für eine Zeitdauer von 5 bis 30 Stunden. EP 018 561 beschreibt die Herstellung von Gerinnungsfaktoren durch Erhitzen auf 60-70 °C in Gegenwart von Glycin und einem Saccharid in Lösung. EP 0 094 611 beschreibt eine FVIII-angereicherte Zusammensetzung zur Herstellung eines Arzneimittels, die in gefriergetrocknetem Zustand bei 60 °C bis 125 °C erhitzt wird, mit einer spezifischen Aktivität größer als 300 FVIII-Einheiten pro Gramm Protein.

In der Zeitschrift "Hemophilia World 1995; Vol. 2; No. 3" sind in einer Beilage zu diesem Heft die zur Zeit in den USA verfügbaren FVIII- und FIX-Konzentrate unter dem Titel "Clotting Factor Concentrates, USA 1995" zusammengefaßt. Aus dieser Publikation sind in Tab. 1 Angaben über die Virusinaktivierungsverfahren, mit denen diese Produkte behandelt werden, entnommen und durch europäische Produkte und Hersteller ergänzt.

**Tab. 1**

| **Faktor VIII Konzentrate (Handelsprodukte 1995)** | | | | | |
|---|---|---|---|---|---|
| lfd. Nr. | Handelsname | Hersteller | Virusinaktivierug | spezifische Aktivität ohne Alb. Endbehälter | |
| 1 | Haemate | Behringwerke | 60 °C, 10 h; flüssig + Stabilisator | ---- | 1-2 |
| 2 | Beriate P | Behringwerke | 60 °C, 10 h; flüssig + Stabilisator | ---- | ca. 100 |
| 3 | Profilate HAT | Alpha | SD; 80 °C, 72 (GT) | ---- | 5-15 |
| 4 | Koate | Miles/Cutter | SD | ---- | 10-20 |
| 5 | Octavi SD Plus | Octapharma | SD; 63 °C, 10 h, flüssig + Stabilisator | ---- | ca. 100 |
| 6 | Emoclot | Aima | SD; 100 °C, 30 Min | ---- | ca. 100 |
| 7 | Haemoctin SDH | Biotest Pharma | SD; 100 °C, 30 Min | ---- | ca. 100 |
| 8 | Immunate | Immuno AG | 60 °C, 10 h; Dampf | ---- | 10 |
| 9 | Alpanate | Alpha | SD | ---- | 20 - 30 |
| 10 | Hemofil M | Baxter | SD | > 500 | 2 - 10 |
| 11 | Monoclate | Armour | 60 °C, 10 h; flüssig + Stabilisator | > 500 | 2 -10 |
| 12 | Octanativ | Kabi | SD | > 500 | ca. 20 |

| | | | | | |
|---|---|---|---|---|---|
| SD = solvent detergent Alb = Albumin GT = gefriergetrocknet | | | | | |

Die in der Tab. 1 aufgeführten FVIII-Konzentrate sind in den letzten zehn Jahren entwickelt worden (Kasper CK, Lusher JM and the Transfusion Practices Committee of the American Association of Blood Banks. "Recent evolution of clotting factor concentrates for hemophilia A and B". Transfusion 1993, 33: 422-4334). Das wichtigste Ziel bei der Entwicklung dieser Produkte war die Verbesserung ihrer Sicherheit bezüglich der Virusübertragung. Dieses Ziel wurde durch die Verbesserung des Spender-Screening und der Verwendung neuer Techniken zur Virusinaktivierung und Aufreinigung des FVIII erreicht.

Ende der 70er und während der 80er Jahre wurden zwei der wichtigsten Virusinaktivierungsverfahren für FVIII-Konzentrate entwickelt. Das eine Verfahren beinhaltet eine Hitzebehandlung des FVIII in Lösung in Gegenwart von Stabilisatoren. Das andere Verfahren beschreibt die Verwendung von Alkylphosphaten. 1979 wurde in der EP 0 018 561 das Erhitzen des FVIII in Lösung in Gegenwart von Zuckern (40-60 %) und Aminosäuren (1-2,5 Mol/l) beschrieben. Das Erhitzen von Proteinen in Lösungen in Gegenwart von Zuckern wurde durch die Zugabe von Ca-lonen-haltigen Salzen erweitert (EP 0 106 269). Ergänzt wurde die Methode der Virusinaktivierung in Proteinlösungen durch Varianten der genannten Veröffentlichungen, wie der EP 0 035 204, in welcher der Temperaturbereich von 60 °C auf 75 °C erweitert wurde und die Konzentration der Zucker von 30 % bis zur Sättigung verwendet wurde. Das Erhitzen von FVIII-haltigen Proteinfraktionen in gefriergetrocknetem Zustand ist in der EP 0 171 506 beschrieben. Der Temperaturbereich umfaßt 60°C bis 125°C. Die Dampfbehandlung von FVIII-haltigen Plasmafraktionen ist bereits oben angeführt worden.

Die Behandlung von FVIII-haltigen Plasmafraktionen mit einer Kombination eines Alkylphosphats und eines Detergens ist in der EP 0 131 740 beschrieben. Dieses Verfahren hat sich besonders wirksam bei der Inaktivierung behüllter Viren erwiesen (Horowitz MS et al.,Lancet 1988, 2: 186-189; Horowitz B, Curr. Stud. Hematol. Blood Transfus. 1989, 56: 83-96; Horowitz B, Transfusion 1985, 25: 516-522). Dieses Virusinaktivierungsverfahren (Solvent-Detergent (SD)-Verfahren) ist jedoch prinzipiell nicht in der Lage, Viren ohne lipidhaltige Virushülle zu inaktivieren.

Da bei der Herstellung von Arzneimitteln aus Plasma vom Menschen die Gefahr der Übertragung von Infektionserregern durch Screening der Spenderplasmen nicht auszuschließen ist, sollte diese Gefahr durch die Anwendung von Virusinaktivierungsverfahren weiter reduziert werden. Maßnahmen zur Inaktivierung der behüllten Viren wurden besonders erforderlich, nachdem 1992/1993 im Zusammenhang mit einem SD-behandeltem FVIII-Konzentrat in ca. 100 Fällen von einer Hepatitis A-Infektion berichtet wurde (Lancet (1992), 340: 123; Lancet (1993), 341: 179; Ann. Intern. Med. (1994); MMWR (1996), Vol. 45, Nr. 2: 29-32). Diese Hepatitis A-Fälle hatten einen Stufenplan deutscher Behörden zur Folge (Pharm. Ind. 1993; 4: 71-72).

Neben der Gefahr der Übertragung von Infektionserregern durch FVIII-Produkte ist die Entstehung von FVIII-Inhibitoren (FVIII-Antikörper) ein zentrales Problem der Substitutionstherapie der Hämophilie A. Antikörper entstehen bei 3-52 % der Patienten mit schwerer Hämophilie A, die durch eine Reihe von Variablen erklärbar sind (De Biasi R, et al., Thrombosis Haemostasis 1994, 71: 544-7; Ehrenforth S, et al., Lancet 1992, 339: 594-8; Scharrer I, et al., Blood Coag Fibrinol 1993, 4: 753-8; McMillan CW, et al., Blood 1988, 71: 344-8). Derartige Variable betreffen das Alter der Patienten, die Häufigkeit der Behandlung und den FVIII-Präparatetyp (Addiego JE, et al., Thromb. Haemost. 1993, 67: 19-27; Burnouf T, et al. Vox. Sang. 1991, 60: 8-15; Peerlinck K, et al., Thromb. Haemost. 1993, 69: 115-8; Rodendaal FR, et al., Blood 1993, 81: 2180-6; Sultan Y, Thromb. Haemost. 1992, 67: 600-2; Peerlinck K, et al., Blood 1993, 81; 3332-5; Guérois C, et al., Thrombos Haemostas 1995, 73 (2): 215-8).

Unterschiede in der Immunantwort der Patienten oder ihre genetische Prädisposition könnten die inhibitor-Izidenz beeinflussen (Roberts HR, Blood Coag and Fibrinol 1991, 2 (1): 21-23; Hoyer L, Brit J of Haematology 1995, 90, 498-501).

Ferner wurde gezeigt, daß verschiedene FVIII-Genmutationen zur Antikörperentstehung korrelieren (Schwaab R, et al., Thrombosis and Haemostasis 1995, 74 (6), 1402-1406).

Die Variablen, die die FVIII-Inhibitor-Inzidenz beeinflussen, sind:
1. Der Patient: Der genetische Defekt, charakteristische immunologische Reaktionen, Immunstatus
2. Die Behandlung: Das FVIII-Produkt, die Zahl der Expositionen
3. Die Beurteilung: Die Überwachung und die Sensitivität der Überwachungsmethoden.

Obwohl bis heute keine eindeutige Zuordnung einer einzigen Ursache für das Entstehen von FVIII-Antikörpern mit Inhibitoreigenschaften möglich ist, zeigen die Ergebnisse einzelner Studien die Bedeutung, die den FVIII-Präparaten bei der Induktion von FVIII-Inhibitoren zukommt (Vermylen I., et al., Acta Clinica Belgica (1991); 46: 20).

Zuvor bereits häufig behandelte Hämophilie A-Patienten (PTPs = previously multitransfused hemophiliacs) wurden von Mai 1990 bis Sept. 1991 mit einem neuen FVIII-Konzentrat behandelt. Dieses Produkt war durch Chromatographie an Glas gereinigt worden und pasteurisiert. Von 47 Patienten, die ausschließlich mit diesem Produkt behandelt wurden, entwickelten 4 einen hochtitrigen Inhibitor (40, 90, 100 und 200 Bethesda Units (BU)). Ein weiterer Patient (20 BU) war im wesentlichen ebenfalls mit diesem FVIII-Produkt behandelt worden. Die Anwendung dieses Produktes wurde aufgrund dieser Befunde beendet.

Die Herstellung eines hochgereinigten FVIII-Konzentrates aus großen Plasmapools mit einer spez. Aktivität von 100 setzt voraus, daß mehrere Anforderungen an das Produktionsverfahren für ein deratiges FVIII-Konzentrat erfüllt werden müssen.

Die Auswirkung der Virusinaktivierungsmaßnahmen auf die Struktur der Proteine ist eine der zentralen Fragestellungen, insbesondere zur Problematik der Inhibitoren. Die wesentlichste biologische Funktion des FVIII, seine Aktivität, sollte im FVIII-Konzentrat, das zur Behandlung der Hämophilen eingesetzt wird, ein nahezu identisches Verhältnis zu dem FVIII-Protein, dem FVIII-Ag, aufweisen, wie in dem Medium; dem Plasma, aus dem er isoliert wird.

Das FVIII-Konzentrat, das nach der Reinigung aus Plasma gewonnen wird, sollte außer dem FVIII im wesentlichen nur noch den von Willebrand-Faktor (vWF) enthalten, sein natürliches Trägerprotein. Da es das Ziel der Bemühungen der Reinigung des FVIII aus Plasma ist, diesen von den übrigen Plasmaproteinen zu trennen, sollte die Verwendung von Plasmaproteinen als Stabilisatoren vermieden werden, da hierdurch die vorhergehenden Bemühungen der Aufreinigung hinfällig werden. Demgemäß sollte der Hämophile nur das von ihm benötigte Protein erhalten. Der gereinigte FVIII aus Plasma besteht aus einer Mischung von Polypeptiden mit Molekulargewichten zwischen 80 bis 210 kDa, die eine Reihe von Untereinheiten einer Serie von Heterodimeren repräsentieren (Kaufmann, R., Transfusion Medicine Reviews 1992, 4: 235-246).

Somit liegt der vorliegenden Erfindung die Aufgabe zugrunde, ein neues Verfahren zur Aufreinigung und Virusinaktivierung Faktor VIII-enthaltender Ausgangsmaterialien, im wesentlichen ohne Verwendung von Stabilisatoren, bereitzustellen.

Diese Aufgabe wird durch das in den Ansprüchen gekennzeichnete Verfahren gelöst. Insbesondere wird ein Verfahren zur Herstellung einer Faktor VIII-enthaltenden Zusammensetzung bereitgestellt, umfassend die Schritte:
(a) Tri(n-butyl)phosphat-/Detergens-Behandlung einer Faktor VIII-enthaltenden Lösung, und
(b) Hitzebehandlung eines gefriergetrockneten FVIII-enthaltenden Materials, das eine Restfeuchte im Bereich von 0,3 bis 1,8 Gew.-% aufweist, bei etwa 100°C für 15 bis 120 min,
wobei die Faktor VIII-enthaltende Lösung vor dem Gefriertrocknen in flüssigem Stickstoff schockgefroren wird.

Das erfindungsgemäße Verfahren zur Herstellung eines nicht immunogen wirkenden hochgereinigten FVIII-Konzentrates aus Plasma vom Menschen wird unter Anwendung von zwei verschiedenen Virusinaktivierungsverfahren hergestellt. Diese doppelte Virusinaktivierung umfaßt, wie vorstehend ausgeführt, die Behandlung der FVIII-Lösung mit Tri-(n-butyl)phosphat ("TNBP")/Polyoxyethylen-Derivat der Sorbitanester (Tween® ) (SD: Solvent/Detergent = Lösungsmittel/-Detergens), vorzugsweise 0,2-0,5 Gew.-% TNBP/0,5-3 Gew.-% Tween® , und eine Hitzebehandlung des gefriergetrockneten FVIII-Endproduktes im Endbehältnis bei 100°C für 30 Minuten, bei einem Restfeuchtegehalt von 0.3 % bis 1,8 Gew.-%. Hinzu kommen vorzugsweise virusabreichernde Schritte bei der Herstellung.

Das erfindungsgemäß hergestellte FVIII-Konzentrat aus Plasma vom Menschen wird im folgenden Text als "FVIII SDH" bezeichnet (SDH = Solvent/Detergent/-Hitze). Der FVIII SDH wird vorzugsweise aus Kryopräzipitat als Ausgangsmaterial hergestellt, das aus einem Plasmapool nach bekannten Methoden gewonnen wird. Im Kryopräzipitat sind nahezu alle Plasmaproteine vorhanden, einige in angereicherter Form gegenüber Plasma, insbesondere der FVIII und Fibrinogen.

Das erfindungsgemäße Verfahren des FVIII SDH umfaßt in einer bevorzugten Ausführungsform folgende Herstellungsschritte:
(1) Die Abtrennung des Fibrinogens und gegebenenfalls die Entfernung weiterer Gerinnungsfaktoren des PPSB-Komplexes erfolgt beispielsweise über eine Ethanolfällung und/oder durch die Adsorption an Al(OH)₃.
(2) Die Virusinaktivierung der FVIII-Rohfraktion erfolgt mit vorzugsweise TNBP/Tween® 80.
(3) Die spezifische Säulenchromatographie an lonenaustauschern führt zur Entfernung von TNBP und Tween® 80, sowie zur Abtrennung der restlichen Plasmaproteine.
(4) Das Eluat wird vorzugsweise dia- und/oder ultrafiltriert, die Elektrolyte werden auf die gewünschten Konzentrationen eingestellt.
(5) Nach der Sterilfiltration erfolgt die Endabfüllung in Glasflaschen, die in flüssigen Stickstoff (etwa - 195°C) getaucht werden, wodurch die FVIIILösung schockgefroren wird.
(6) Die Gefriertrocknung der gefrorenen FVIII-Lösung erfolgt im Hochvakuum und wird bis zu einer Restfeuchte des Endproduktes im Bereich von 0,3 bis 1,8 % ausgeführt.
(7) Das gefriergetrocknete Präparat wird für 30 min im Autoklaven auf 100°C erhitzt.

Das erfindungsgemäß hergestellte FVIII-Produkt wirkt nicht immunogen und ist gegenüber dem Ausgangsplasma beispielsweise um das etwa 10⁴-fache, insbesondere durch die Anwendung chromatographischer Reinigung, angereichert.

Die virusabreichernden Schritte der FVIII-Aufarbeitung und Reinigung aus Plasma vom Menschen, unter Anwendung des erfindungsgemäßen Verfahrens zur Herstellung eines nicht immunogen wirkenden FVIII-Konzentrates sind in Tab. 2 aufgeführt. Für einige relevante behüllte und nicht-behüllte Viruen sind die Inaktivierungsraten für jeden Aufarbeitungsschritt der FVIII-Konzentrat-Herstellung in log 10 aufgeführt.

**Tab. 2**

| **Virusabreicherung und Vrusinaktivierung für nicht immunogen wirkenden FVIII aus Plasma** | | | | | | | |
|---|---|---|---|---|---|---|---|
| **(Virus-Reduktion: log 10)** | | | | | | | |
| Schritt/Behandlung | | | | | | | |
| Virus | Neutalisation | Kryopr 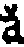 zip. | Al(OH)₃ | TNBP Tween | Chromatograph. | 100 °C 30 min | gesamter Reduktionsfaktor |
| HIV-1 | | 1.3 | 0.4 | > 6.4 | 1.9 | > 6.6 | > 16.2 |
| PSR | | 3.2 | 1.1 | >6.8 | 1.1 | > 4.6 | > 16.8 |
| VSV | | 1.5 | 0.5 | >4.7 | 0.6 | >5.8 | >11.9 |
| BVDV | | 1.2 | n.t. | > 6.8* | 1.1 | > 6.6 | > 15.7 |
| Sindb. | | n.t.*** | 3.5 | >4.5 | 1.0 | n.t. | > 9.0 |
| Polio | | n.t. | 5.0 | n.t. | 2.2 | 5.0 | 12.2 |
| Parvo** | | | 1.3 | 0.5 | 2.4 | 1.4 | 5.1 |
| Reo | | | 1.6 | n.t. | | > 6.0 | > 7.6 |
| HAV | 4.4 | | 4.1 | | | > 5.3 | > 13.8 |

| | | | | | | | |
|---|---|---|---|---|---|---|---|
| * HCV: > 4.5 log 10 (Schimpansen Studie) ** bovines Parvovirus *** nicht getestet | | | | | | | |

Das nach dem erfindungsgemäßen Verfahren hergestellte FVIII-Konzentrat aus Plasma vom Menschen enthält den FVIII beispielsweise in einer gegenüber dem Plasma 10.000-fach angereicherten Menge. Die pro 1000 IE FVIII SDH (IE = Internationale Einheiten) nachweisbare Konzentration einzelner Proteine ist derjenigen in einem Liter Plasma vorhandenen Proteinkonzentration gegenübergestellt (Tab 3.).

**Tab. 3 Proteinkonzentration im Plasma und FVIII SDH (Durchschnittswerte)**

| Protein | Plasmakonzentration | FVIII SDH | Methode |
|---|---|---|---|
| | (mg/l) | (mg/1000 IE) | |
| FVIII | 0.1-0.2 | 0.1-0.2 | 1 |
| Gesamtprotein | 65 000 | 10 | 2 |
| vWF | 18 | 6 | 1 |
| Fibrinogen | 3 000 | 3.5 | 3 |
| Fibronectin | 30 | 0.3 | 3 |
| Albumin | 44 000 | 0.03 | 3 |
| IgG | 12 000 | 0.5 | 3 |
| IgM | 1 400 | 0.2 | 3 |
| IgA | 2100 | <0.01 | 3 |

| | | | |
|---|---|---|---|
| 1 = Elisa (Enzyme linked immunosorbent assay) 2 = Bradford (Autor der Methode) 3 = RID (Radiale Immundiffusion) (Bradford MM: Analytical Biochemistry 1976, 72: 248 - 254) | | | |

In dieser Darstellung der Konzentrationsangaben der im FVIII SDH nachweisbaren Proteine ist abzulesen, daß beispielsweise 96 % der im Produkt nachweisbaren Proteinmenge aus FVIII, dem vWF und nicht gerinnbarem Fibrinogen bestehen. Das Fibrinogen liegt ais nicht gerinnbares Fibrinogen vor, d. h. die Fibrinogenbestimmung nach Clauss ergibt ein negatives Ergebnis (Clauss A, Acta Haemat 1957, 17: 237 - 246).

Die Methoden, die zur Herstellung der FVIII-Konzentrate und zur Inaktivierung bzw. Eliminierung im Blutplasma vorkommender Viren angewandt werden, haben möglicherweise einen Einfluß auf die Struktur und biologische Aktivität der Proteine. Eine Grundvoraussetzung der Anwendbarkeit der FVIII-Produkte ist, daß sie sich vor ihrer Anwendung nach der Zugabe des Lösungsmittels - normalerweise ist dies ein für klinische Anwendungen geeignetes Wasser (*aqua ad injectabilia*) *-* rückstandlos auflösen. Denaturierte Proteine sind in Wasser im wesentlichen unlöslich. Die Struktur und biologische Aktivität des erfindungsgemäß hergestellten FVIII-Produktes bleibt erhalten, wenn dieser nach dem erfindungsgemäßen Verfahren hergestellt wird, da sich nur unter diesen Bedingungen das FVIII-Konzentrat auch nach dem Erhitzen, bis zu 2 Stunden auf 100 °C, rückstandslos mit *aqua ad injectabilia* auflöst.

Eine entscheidende Voraussetzung dafür, daß der erfindungsgemäße FVIII SDH für 30 min auf 100°C erhitzbar ist, ohne dabei denaturiert zu werden und sich trotzdem rückstandslos mit Wasser lösen läßt, ohne daß dem Gefriertrocknungs-Puffer ein Stabilisator in Form von Proteinen oder Zuckern zugesetzt werden muß, ist das Schockeinfrieren der FVIII-Lösung in flüssigen Stickstoff bei einer Temperatur von -195°C vor der Gefriertrocknung. Das nach dem erfindungsgemäßen Verfahren hergestellte FVIII-Konzentrat FVIII SDH wird überraschenderweise in seiner biologischen Struktur und biologischer Aktivität nicht verändert und eine Denaturierung der Proteine durch die Erhitzung findet nicht statt.

Zur Überprüfung der Auswirkung des erfindungsgemäßen Herstellungsverfahrens auf die Struktur der Proteine wurde eine Reihe physikalischer und biologischer Tests durchgeführt (Kotitschke R et al, Hämostaseologie 1994, 14: 100-3; Arrighi S et al., Thromb Haemost 1995, 74 (3); 868 -73). Insbesondere ist die Auswirkung der Hitzebehandlung auf die Proteine unter den Bedingungen des erfindungsgemäßen Herstellungsverfahrens überprüft worden, da der FVIII bekanntermaßen extrem lagerinstabil und empfindlich gegenüber der Hitzebehandlung ist.

Eine Hitzebehandlung des FVIII in Lösung erfordert üblicherweise die Stabilisierung des FVIII durch die Zugabe bestimmter Stabilisatoren. Eine Hitzebehandlung des FVIII in gefriergetrocknetem Zustand ohne nennenswerten Aktivitätsverlust ist ferner nur für FVIII-Produkte bekannt, deren spezische Aktivität nach der Herstellung aus Plasma sehr niedrig ist (z.B. < 20) oder denen Stabilisatoren in Form von Proteinen oder Zucke zugesetzt werden müssen (s. Tab. 1).

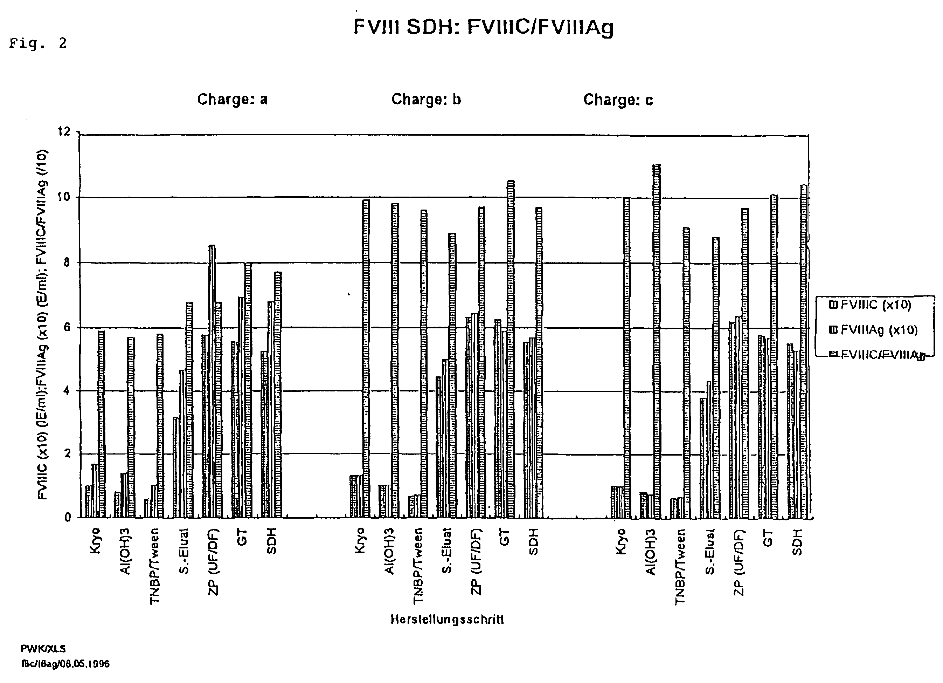

Das erfindungsgemäße FVIII SDH-Produkt kann überaschenderweise ohne nennenswerten Aktivitätsverlust bei 100 ° C bis zu 2 Stunden erhitzt werden, wenn der Restfeuchtegehalt weniger als 1,8 % beträgt und die Faktor VIII - enthaltende Lösung vor dem Gefriertrocknen in flüssigem Stickstoff schockgefroren wird. Aus den im folgenden aufgeführten Daten wird ersichtlich, daß das erhitzte und nicht-erhitzte FVIII Produkt bei der Überprüfung der Struktur und Aktivität identische Ergebnisse ergab (Fig. 2 und Tab. 4).

**Tab. 4**

| **Haemoctin (R) SDH: FVIIIC/FVIIIAg** | | | |
|---|---|---|---|
| Behandlung | FVIIIC (x10) | FVIIIAg (x10) | FVIIIC/FVIIIAg (/10) |
| Kryo | 0,98 | 1,66 | 5,9 |
| Al(OH)3 | 0,79 | 1,38 | 5,7 |
| TNBP/Tween | 0,59 | 1,01 | 5,8 |
| S.-Eluat | 3,18 | 4,68 | 6,8 |
| ZP (UF/DF) | 5,79 | 8,55 | 6,8 |
| GT | 5,57 | 6,95 | 8 |
| SDH | 5,25 | 6,8 | 7,7 |
| | | | |
| | | | |
| Kryo | 1,29 | 1,3 | 9,9 |
| Al(OH)3 | 0,99 | 1,01 | 9,8 |
| TNBP/Tween | 0,67 | 0,7 | 9,6 |
| S.-Eluat | 4,46 | 5 | 8,9 |
| ZP (UF/DF) | 6,31 | 6,45 | 9,7 |
| GT | 6,25 | 5,9 | 10,5 |
| SDH | 5,55 | 5,7 | 9,7 |
| | | | |
| | | | |
| Kryo | 0,98 | 0,98 | 10 |
| Al(OH)3 | 0,81 | 0,73 | 11 |
| TNBP/Tween | 0,61 | 0,67 | 9,1 |
| S.-Eluat | 3,8 | 4,34 | 8,8 |
| ZP (UF/DF) | 6,2 | 6,38 | 9,7 |
| GT | 5,78 | 5,7 | 10,1 |
| SDH | 5,52 | 5,3 | 10,4 |

Die Figuren zeigen:

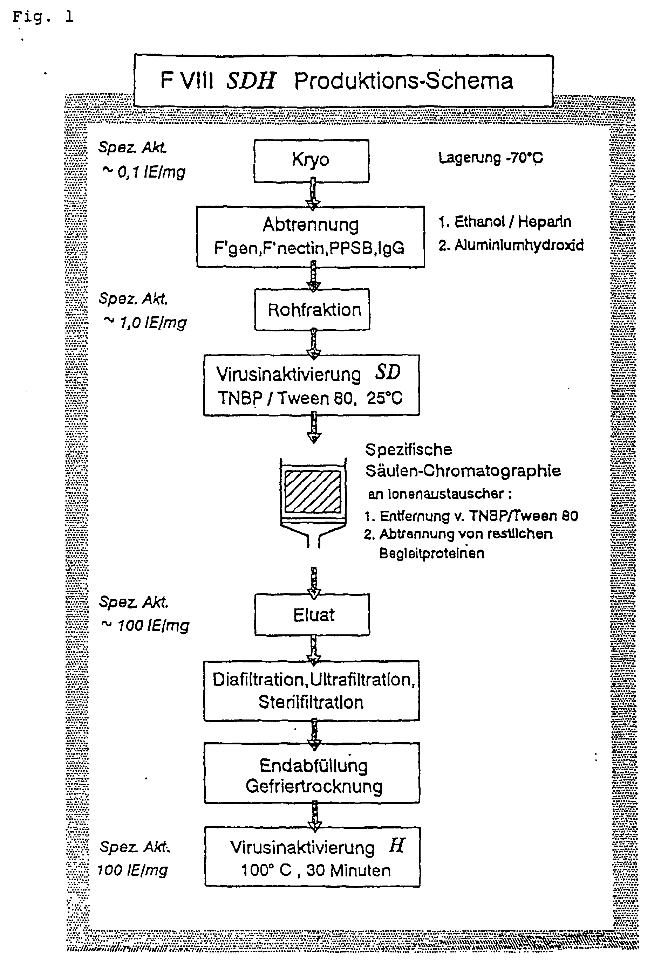

Fig. 1 zeigt schematisch die FVIII SDH-Herstellung in einer bevorzugten Ausführungsform.
Fig. 2 zeigt das Verhältnis von FVIIIC/FVIIIag an verschiedenen FVIII-Herstellungsschritten von drei FVIII SDH-Chargen. Die Charge a unterscheidet sich von den Chargen b und c deutlich durch das verminderte Verhältnis des Ausgangsproduktes der FVIII-Herstellung des Kryos. Um auf dieser Grafik die Säulen für die einzelnen Parameter in nahezu vergleichbarer Größe darstellen zu können, wurden die einzelnen Parameter entweder mit 10 multipliziert oder durch 10 dividiert. Das Verhältnis muß in dieser Darstellung durch 10 dividiert werden, damit erkennbar wird, daß sie für die Chargen b und c an jedem Herstellungsschritt nahe = 1,0 ist. Das Kryo der Charge a weist ein Verhältnis von etwa 0,6 auf, ein Hinweis auf verlorengegangene FVIII-Aktivität bei der Herstellung und Lagerung dieser Kryo-Charge. Der Aufarbeitungsprozeß zum hochgereinigten FVIII-Konzentrat bringt das Verhältnis auf 0,8, aber nicht mehr auf 1,0. Entscheidend bei dieser Studie ist jedoch der Vergleich des Verhältnisses der erhitzten und nicht-erhitzten Proben. Diese ist für beide Produkte nahezu gleich und bestätigt damit den relativ geringen Aktivitätsverlust durch die Hitzebehandlung.
   Die Auswirkung des erfindungsgemäßen Herstellungsverfahrens auf die Proteinstruktur wurde anhand der Molekulargewichtsverteilung und SDS-Polyacrylamid-Gelelektrophorese (PAGE) überprüft. Zusätzlich wurde das nicht-erhitzte (FVIII SD) und erhitzte Produkt (FVIII SDH) auf die Entstehung von Neoantigenen überprüft (R Kotitschke et al., Hämostaseologie 1994; 14: 100-3; S. Arighi et al., Thromb Haemost 1995; 73 (3): 868-73). Diese Überprüfungen ergaben eine Identität für den FVIII SD und den FVIII SDH, womit gezeigt war, daß die Proteine in dem erfindungsgemäß hergestellten FVIII-Konzentrat in ihrer Proteinstruktur unverändert waren.

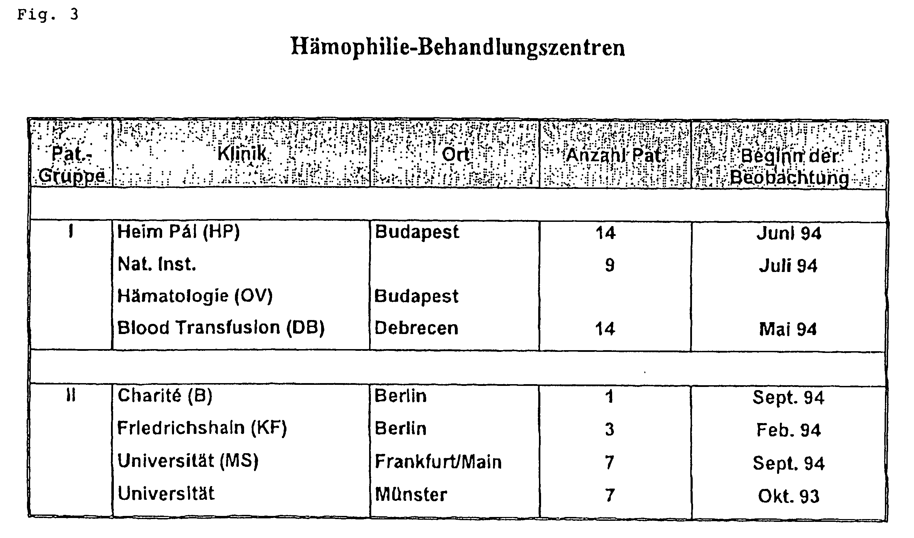

Fig. 3 zeigt die Anwendungsbeobachtung für FVIII SDH. Die Anwendungsbeobachtung an sieben Behandlungszentren für Hämophilie A wurde 1993 begonnen. 51 Patienten wurden in diese Anwendungsbeobachtung einbezogen, mit der längsten Beobachtungszeit von 27 Monaten. In Ungarn sind vor 1993 nahezu alle Hämophilie A-Patienten mit Kryopräzipitat und/oder nur mit einem FVIII-Konzentrat behandelt worden. Daher war es möglich, die Inhibitor-Inzidenz an einer relativ homogenen Patientengruppe nach der Behandlung mit FVIII SDH zu prüfen und die Inhibitor-Inzidenz dieser Gruppe (Gruppe I) zu vergleichen, die an Behandlungszentren für Hämophilie A in Deutschland (Gruppe II) mit verschiedenen FVIII-Konzentraten vorbehandelt waren. Bei keinem der 51 Patienten ist ein Inhibitor aufgetreten. Die Patienten wurden über einen Zeitraum von 4 bis 26 Monaten untersucht, die überwiegende Anzahl über einen Zeitraum von 12 Monaten. Die maximale Anzahl der kumulativen Behandlungstage betrug 349. Ein Behandlungstag umfaßt die FVIII-Menge, die zur Behandlung einer Blutungsepisode verwendet wurde.

Die "response", "*in vivo* recovery" und Halbwertszeit wurde mit dem FVIII 1-StufenTest und einem chromogenen Substrat (an 16 Patienten) bestimmt; vgl. Tab. 5. Diese Daten entsprechen denen in der Vergangenheit von anderen Autoren publizierten für pd FVIII-Konzentrate (pd = plasma derived = aus Plasma hergestellt).

**Tab. 5**

| **FVIII SDH: Biologische Halbwertszeit und in vivo recovery an 16 Hämophilie A-Patienten** | | |
|---|---|---|
| | **1-Stufen-Test** | **chromogenes Substrat** |
| in vivo recovery (%) | 71 ± 15 | 77 ±17 |
| Response (%) | 1.6 ± 0.4 | 1.7 ±0.4 |
| Halbwertszeit | 13.0 ± 2.8 | 12.7 ±3.2 |

Das erfindungsgemäße Verfahren wird durch die nachstehenden Beispiele näher erläutert.

### Beispiel 1

Herstellung von FVIII aus Plasma vom Menschen, der nicht immunogen nach der Substitution an Patienten ist.

Spenderplasma: Neun Teile Spender-Venenblut wurden zu einem Teil einer 3,8%igen Natriumcitrat-Stabilisatorlösung gegeben. Das Blut wurde direkt nach der Blutentnahme zentrifugiert und in die in physiologischer Kochsalzlösung aufgeschlemmten Erythrozyten dem Spender reinfundiert (Plasmapherese). Das Plasma wurde spätestens innerhalb von 18 Stunden eingefroren.

Das gefrorene Plasma wurde bei einer Temperatur von +2°C bis +4°C aufgetaut. Die Plasmen von mehreren Spendern wurden gepoolt (gemischt). Die Kälteausfällung (Kryopräzipitat) wurde in an sich bekannter Weise durch Zentrifugation gewonnen.

1 kg Kryopräzipitat, gewonnen aus ca. 100 l Plasma, wurde zu 3 kg einer Wasser/Ethanol-Mischung (10 g Ethanol auf 1 l H₂O), die 900 IE Heparin enthielt, suspendiert.

Der pH-Wert der Suspension wurde unter Rühren mit 0,1 M Essigsäure auf pH 7,0 eingestellt. Die Mischung wurde auf Zimmertemperatur erwärmt und für 30 Minuten gerührt. Zu der so erhaltenen Lösung wurden 108 g einer 2 % Al(OH)₃₋Suspension pro kg eingesetztes Kryo zugesetzt und für 15 min bei Zimmertemperatur gerührt. Der pH-Wert wurde durch Zugabe von 0,1 M Essigsäure auf pH 6,55 eingestellt und die Temperatur auf 15°C abgekühlt. Der Niederschlag wurde abzentrifugiert und verworfen.

Der Überstand wurde zur Virusinaktivierung mit einer Mischung aus Tri-(n-butyl) phosphat (TNBP) (0,3 Gew.-%) und Tween® 80 (1 Gew.-%) behandelt. Pro 1 kg der FVIIILösung wurden 10,52 g Tween® 80 und 3,16 ml TNBP benötigt. Das Tween® 80 wurde mit 4 ml dest. H₂O gelöst. Der pH-Wert der FVIII-Lösung wurde mit 1 M NaOH auf pH 7,1 eingestellt und die Lösung auf 25°C vorgewärmt. Die Mischung aus TNBP und Tween® 80 wurde über einen Zeitraum von 15 Minuten unter Rühren zugegeben, Die mit TNBP/Tween® 80 versetzte FVIII Lösung wurde für 13 Stunden bei etwa 25°C gerührt. Die FVIII-Lösung wurde anschließend an einem Anionenaustauschergel chromatographiert. Die Säule wurde vor dem Auftragen der Proteinlösung mit einem Puffer von pH 6,95 aus NaCl (0,12 M), Na-Citrat (0,01 M), Glycin (0,12 M) und CaCl₂ (0,001 M) äquilibriert. Die Extinktion des Säuleneluates wurde bei 280 nm gemessen. Die sterilisierte FVIII-Lösung wurde auf das äquilibrierte Gel aufgetragen und mit dem Äquilibrierungspuffer nachgewaschen, bis das UV-Signal die Basislinie erreicht hatte. Zur Entfernung von Begleitproteinen wurde die Säule mit einem Puffer erhöhter Ionenstärke aus NaCl (0,16 M), Na-Citrat (0,01 M), Glycin (0,12 M) und CaCl₂ (0,001 M) bei pH 6,95 gewaschen.

Die Elution des FVIII wurde mit folgendem Elutionspuffer ausgeführt: 0,25 M NaCl, 0,01 M Na-Citrat, 0,12 M Glycin, 0,001 M CaCl₂ bei pH 6,95. Das FVIII-haltige Eluat der Chromatographie wurde mit einer Ultrafiltration auf etwa die Hälfte der eingesetzten Eluatvolumen ankonzentriert und mit einem Puffer aus 0,01 M NaCitrat, 0,12 M Glycin und 0,001 M CaCl₂ bis zu einer FVIII-Aktivität der Lösung von etwa 110 IE FVIII pro ml verdünnt. Die FVIII-Lösung wurde sterilfiltriert und à 10 ml in die Endbehältnisse (20 ml Glasflaschen) abgefüllt. Die Glasflaschen mit der FVIII-Lösung wurden bei -195°C in flüssigen Stickstoff getaucht und anschließend in einer Gefriertrocknungsanlage bis zu einer Restfeuchte des FVIII-Produktes von etwa 0,5 % getrocknet.

Die Flaschen mit dem gefriergetrockneten Produkt wurden in einen Autoklaven gestellt und für 30 Minuten auf 100°C erhitzt. Die Temperatumessung erfolgte mit dem Gerät "Datatrace Micropack" der Fa. Hero Instruments Electronic Vertriebs GmbH, Deutschland. Direkt nach der Entnahme der Flaschen aus dem Autoklaven wurden sie in einen Kühlraum (ca. +4°C) zum Abkühlen eingebracht. Die FVIII-Aktivität der erhitzen Probe betrug 95 % der nicht-erhitzten Probe.

### Beispiel 2

1 kg Kryopräzipitat wurde in der gleichen Weise wie in Beispiel 1 hergestellt und gelöst. Die Aufarbeitung des Kryopräzipitates bis zur Ultrafiltration wurde in gleicher Weise wie in Beispiel 1 beschrieben durchgeführt. Die Verdünnung der ankonzentrierten FVIII-Säulen-Eluatlösung erfolgte mit dem Puffer aus 0,01 M Na-Citrat, 0,12 M Glycin und 0,01 M CaCl₂ bis zu einer FVIII-Aktivität von 50 IE FVIII pro ml. Die FVIII-Lösung wurde sterilfiltriert und à 10 ml in die Endbehätnisse (20 ml Glasflaschen) abgefüllt. Das Einfrieren dieser Flaschen, sowie die Gefriertrocknung und die Hitzebehandlung erfolgte wie unter Beispiel 1 beschrieben.

## Patentansprüche

1. Verfahren zur Herstellung einer nicht-immunogenen Faktor VIII-enthaltenden Zusammensetzung, umfassend die Schritte:
(a) Tri(n-butyl)phosphat-/Detergens-Behandlung einer Faktor VIII-enthaltenden Lösung,und
(b) Hitzebehandlung eines gefriergetrockneten FVIII-enthaltenden Materials, das eine Restfeuchte im Bereich von 0,3 bis 1,8 Gew.-% aufweist, bei etwa 100°C für 15-120 min,
wobei die Faktor VIII-enthaltende Lösung vor dem Gefriertrocknen in flüssigem Stickstoff schockgefroren wird.

2. Verfahren nach Anspruch 1, wobei das Detergens ein Polyoxyethylen-Derivat eines Sorbitanesters ist.

3. Verfahren nach Anspruch 1 oder 2, worin vor Schritt (a) Fibrinogen und gegebenenfalls weitere Gerinnungsfaktoren des PPSB-Komplexes durch eine Ethanolfällung und/oder eine Adsorption an Al(OH)₃ aus der FVIII-enthaltenden Lösung entfernt werden.

4. Verfahren nach einem der Ansprüche 1 bis 3, worin nach Schritt (a) das Tri(n-butyl)phosphat und das Detergens durch lonenaustauschchromatographie aus der FVIII-enthaltenden Lösung entfernt werden.

5. Verfahren nach Anspruch 4, worin die FVIII-enthaltende Lösung als Eluat der lonenaustauschchromatographie dia- und/oder ultrafiltriert wird.

6. Verfahren nach Anspruch 5, worin die FVIII-enthaltende Lösung nach der Dia- und/oder Ultrafiltration sterilfiltriert wird.

7. Verfahren nach einem der Ansprüche 1 bis 6, worin die Hitzebehandlung in einem Autoklaven durchgeführt wird.

8. Nicht-immunogene FVIII-enthaltende Zusammensetzung, erhältlich durch ein gemäß einem der Ansprüche 1 bis 7 gekennzeichneten Verfahren.

## Claims

1. Process for the preparation of a non-immunogenic composition containing factor VIII, comprising the following steps:
(a) treatment of a solution containing factor VIII with tri-n-butyl phosphate/detergent, and
(b) heat treatment of a freeze-dried FVIII-containing material, with a residual moisture content ranging from 0.3 to 1.8 wt.%, at about 100°C for 15-120 min.
wherein, prior to freeze drying, the solution containing factor VIII is quick-frozen in liquid nitrogen.

2. Process according to Claim 1 wherein the detergent is a polyoxyethylene derivative of a sorbitan ester.

3. Process according to Claim 1 or 2 wherein, prior to step (a), fibrinogen and optionally other clotting factors of the PPSB complex are removed from the FVIII-containing solution by ethanol precipitation and/or adsorption onto Al(OH)₃.

4. Process according to one of Claims 1 to 3 wherein, after step (a), the tri-n-butyl phosphate and the detergent are removed from the FVIII-containing solution by ion exchange chromatography.

5. Process according to Claim 4 wherein the FVIII-containing solution is diafiltered and/or ultrafiltered as the eluate of the ion exchange chromatography.

6. Process according to Claim 5 wherein the FVIII-containing solution is sterile-filtered after the diafiltration and/or ultrafiltration.

7. Process according to one of Claims 1 to 6 wherein the heat treatment is carried out in an autoclave.

8. Non-immunogenic FVIII-containing composition obtainable by a process characterized according to one of Claims 1 to 7.

## Revendications

1. Procédé de préparation d'une composition contenant le facteur VIII, non immunogène, comprenant les étapes :
(a) traitement par phosphate de tri(n-butyle)/détergent d'une solution contenant le facteur VIII, et
(b) traitement thermique d'un matériau contenant le FVIII lyophilisé, qui présente une humidité résiduelle dans la plage de 0,3 à 1,8% en poids, à environ 100°C pendant 15-120 minutes,
la solution contenant le facteur VIII étant rapidement congelée dans l'azote liquide avant lyophilisation.

2. Procédé selon la revendication 1, où le détergent est un dérivé du polyoxyéthylène d'un ester de sorbitan.

3. Procédé selon la revendication 1 ou 2, où avant l'étape (a), on élimine de la solution contenant le FVIII, le fibrinogène et, le cas échéant, d'autres facteurs de coagulation du complexe PPSB par une précipitation à l'éthanol et/ou une adsorption sur Al(OH)₃.

4. Procédé selon l'une quelconque des revendications 1 à 3, où après l'étape (a), on élimine le phosphate de tri(n-butyle) et le détergent par chromatographie échangeuse d'ions de la solution contenant le FVIII.

5. Procédé selon la revendication 4, où la solution contenant le FVIII comme éluat de la chromatographie échangeuse d'ions, est dia- et/ou ultrafiltrée.

6. Procédé selon la revendication 5, où la solution contenant le FVIII est filtrée de manière stérile après la dia- et/ou l'ultrafiltration.

7. Procédé selon l'une quelconque des revendications 1 à 6, où le traitement thermique est réalisé dans un autoclave.

8. Composition contenant le FVIII, non immunogène, pouvant être obtenue à l'aide d'un procédé caractérisé selon l'une quelconque des revendications 1 à 7.
